# EUROPEAN PATENT APPLICATION

(11) **EP 1 939 216 A1**
(43) Date of publication of application: **02.07.2008**
(21) Application number: 07747859.2
(22) Date of filing: 16.03.2007
(51) Int. Cl.: C07K 14/52, A61K 38/19

(54) **IMMUNOMODULATING COMPOSITION**

(30) Priority: 27.03.2006 RU 2006110507
(71) Applicant: Smirnov, Mikhail Nikolaevich, St. Petersburg 191025 (RU)
(72) Inventor: NIKOLAEVA, Zoya Kalenikovna, St.Petersburg, 190000 (RU); YAKOVLEVA, Vera Sergeevna, St.Petersburg, 191014 (RU)
(74) Representative: Glawe, Delfs, Moll
(86) International application number: PCT/RU2007/000129
(87) International publication number: WO 2007/111535

(57) **Abstract**

The inventive composition comprises the water-insoluble fraction of cells of the yeast producer of a heterologous hydrophobic cytokine protein, solubilzer and water, wherein a target hydrophobic cytokine protein has been synthesized in said producer. In particular the cytokine protein is interleukine-2 (IL-2) and the yeast producer is the Saccharomyces cerevisiae strain deposited as No Y-791in the All-Russian Collection of Industrial Microorganisms. Said composition exhibits an immunomodulating activity and can be used in immune-oriented therapy and, in particular, for treating oncologic, infectious, autoimmune and allergic diseases. A composition comprising the water-insoluble fraction of destroyed cells of the heterologous hydrophobic protein-cytokine yeast producer, in which the target hydrophobic protein-cytokine has been synthesised, is also disclosed. Said composition is not active itself and can be used for producing the immunomodulating composition by adding the solubilzer and water thereto.

## Description

The invention relates to immunomodulating drugs and can be used in medicine and veterinary medicine as well. Particularly, the invention relates to immunomodulating compositions that can be used for the purposes of immune-oriented therapy and, in particular, for treatment of oncological, infectious, autoimmune and allergic diseases.

Disability of immune system to effectively perform its basic functions results in development of various kinds of secondary immunodeficiency, which manifests itself clinically through several typical syndromes: infectious, allergic (atopic), autoimmune and lymphoproliferative.

It is of general knowledge for today that major part of frequent diseases and functional disorders is accompanied by immunodeficiencies. Immune suppression is concomitant not only to AIDS, but also to a number of infectious and oncological diseases, traumas, burns and injuries. It is observed at septic conditions of various etiologies, ageing, childbirth, prematurity of newborns and surgical interventions.

The use of means of immune-oriented therapies is an accent of today applied medicine. Treatment of many diseases is impossible without these means and they constitute an obligatory component of complex therapies. Range of drugs having immunotropic activity is rather wide and diverse. From the background art (patent US 4 138 479 published at 06.02.1979) known is water soluble immunopotentiating agent which represents an extraction product of destroyed yeast cell walls.

Known is an immunomodulating composition (patent US 6 509 313, published at 21.01.2003), comprising an agent with cytokine activity, which may represent natural, recombinant or mutated cytokine. The composition is used for stimulating, maintaining or inhibiting the immune response.

The specification of the patent US 6 509 313 mentions a possibility of oral administration of the composition comprising an agent with cytokine activity. However, as it may be concluded form the examples provided, the composition was administered by subcutaneous injections or with use of plaster or inhalator. It is, moreover, mentioned to be advisable "to avoid the hydrolyzing environment of gastrointestinal tract" (last but one paragraph in Column 15).

It should be also mentioned that agents with cytokine activity known so far are highly purified natural or recombinant proteins with extremely high production costs. Composition comprising as the active ingredient the fraction of yeast cell walls is also known (patent US 6 919 082, published at 19.07.2005). It is administered orally and manifests a therapeutic effect, particularly, in the treatment of allergic diseases. The fraction mentioned above stimulates synthesis of short chain fatty acids, whose contribution to healthy condition of human intestines has been recently fairly appreciated. The composition does not utilize the large potential of yeasts as producers of heterologous proteins.

The object of the present invention is to provide a low cost preparation, which can be used for immune-oriented therapy, and, in particular, for the treatment of oncological, infectious, autoimmune and allergic diseases and can be administered to a patient without interrupting the skin integument. Taking into account that the immune system of the patients due to unknown reasons does not recognize the foreign agent which is already present and causes the disease, the authors of the invention have the goal to develop the preparation that would allow providing additional antigen stimulation in order the first stage of immune response to be implemented, with further stimulation of various immune cells proliferation. Thus, one further object of the invention is to provide a pharmaceutical composition comprising factors of activation and proliferation of human and animal immune cells.

This object is solved by providing a composition comprising water insoluble destroyed cell fraction of yeast producer of heterologous hydrophobic cytokine protein, wherein in the producer the target hydrophobic cytokine protein has been synthesized. In particular, the cytokine protein is interleuikin-2 (IL-2), and the yeast producer is *Saccharomyces cerevisiae* strain deposited as No. Y-791 in the All-Russian Collection of Industrial Microorganisms.

Such composition is not active itself, but can be used for immunomodulating purposes after a solubilzer and water is added thereto finally leading to obtainment of the inventive immunomodulating composition.

In an alternative and rather most preferred embodiment the inventive composition comprises the water insoluble destroyed cell fraction of yeast producer, in which the target heterologous hydrophobic cytokine protein, in particular, interleukin-2 (IL-2) has been synthesized, and a solubilzer, in particular, sodium dodecyl sulphate. To activate the composition it is enough to add water.

Finally, the object of the invention is solved by providing a composition comprising water insoluble destroyed cell fraction of heterologous hydrophobic cytokine protein yeast producer, a solubilzer and water, in which producer the target hydrophobic cytokine protein has been synthesized. In particular, the composition is provided comprising a water insoluble destroyed cell fraction of yeast producer, in which producer the heterologous interleukin-2 (IL-2) has been synthesized, and also comprising a solubilzer and water. This composition has immunomodulating activity.

In a preferred embodiment the yeast producer is the *Saccharomyces cerevisiae* strain deposited as No. Y-791 in the All-Russian Collection of Industrial Microorganisms, which strain is the producer of human recombinant interleukin-2. The solubilzer used in the composition is preferably sodium dodecyl sulphate.

The claimed composition allows utilizing the therapeutic potential of heterologous cytokine yeast producer in most efficient way as well as avoiding the very complex procedure of cytokine purifying, which significantly reduces the cost of the obtained composition, obtaining thereby the preparation that can be administered without interrupting the skin integument, namely, orally.

The claimed composition is quite a new approach to immunomodulating drugs. It combines therapeutical effects of yeasts and cytokines, and moreover, the proposed solution, although seems to be very simple, is absolutely unexpected in the present technical field. Providing such a composition may drastically change the approach to cytokine therapy as the composition obtained in such a natural way allows to solve several problems at once, including reduction of cytokine therapy costs, simplifying the drug administration (orally rather than subcutaneously or intravenously), increasing the therapy efficiency, especially in cases of resistance to cytokine monotherapy.

As it may be concluded from the results of the trials of the inventive composition, the composition is highly competitive in its efficiency with well-known cytokine preparations, in particular, with the interleukin-2 preparations and besides that, according to the preliminary data, the water insoluble fraction of destroyed yeast cells represents the activation factor without which the proliferation factor represented by cytokine, in particular, by interleukin-2, does not provide any therapeutic effect.

Within the context of this description three types of influence on immune system are understood as immunomodulation, namely: immunoprotection, immunocorrection and immunorestoration.

Immunoprotection means preventing the development of immune deficiency as a component of multiple organ failure in case of such extraordinary factors or situations as heavy mechanical trauma, extensive surgical intervention, extensive burns, etc. In these cases the immune deficiency is to a large extent associated with improper functioning of inflammation generalizing systems, as well as with blood loss, shock, hypoxia, and endotoxicosis, and also it turns out to be a key component of pathogenesis of acute developing severe multiple organ failures, i.e., life-threatening conditions frequently resulting in lethal outcome. Immunocorrection means the compensation of revealed immune deficiency associated with cellular component of immune system, and elimination of disbalance of this component. Immunorestoration means the reconstruction of the entire pool of the immune competent cells, primarily the T- lymphocytes, and also the restoration of morphological and functional integrity of immune system.

Normally, the immune response is activated in an organism due to penetration of viral, bacterial or mycotic agents and also due to formation of malignant cells in organism, that is, the immune response is activated by antigen. However, physicians frequently face the situations when due to unknown reasons the organism of a patient does not respond to antigen stimulation, and chronic disease develops in the patient concomitant with permanent carriage of a foreign agent (such diseases are tuberculosis, hepatitis B and C, sinusitis, recurrent allergic diseases, chronic Chlamidia and mycoplasmosis, chronic viral infection carrier state, persistent pyodermatitis, pyelonephritis, mycotic infections, etc.) accompanied by immunodeficiency state.

Thus, it may be concluded that use of the proliferation factor solely for immunomodulation purposes is in some cases insufficient and the activation factor is also required.

Particularly, the mechanism of action of the endogenous IL-2 includes stimulation of antigen-dependent proliferation of CD4+ and CD8+ lymphocytes. In other words, successful proliferation of the above mentioned T-lymphocytes and subsequent start of the cascade of immunological events (secretion of numerous cytokines, expression of appropriate receptors, immune response formation, etc.), require both presence of activation factor (antigen stimulation) and proliferation factor (interleukin-2) (Smith K. A. "T-cell growth factor". // Immunol. Rev. - 1980. - 51. - p. 337-357. Rubin J.T. "Interleukin-2: its biology and clinical application in patients with cancer". // Cancer investigation. - 1993. -11 (4). - p. 460-472).

As stated above, depending on the clinical pathology the immune response activation by an antigen can take place resulting from viral, bacterial and mycotic agents invasion of the organism and also due to formation of malignant cells in the organism.

If IL-2 is introduced in the organism under such conditions, it will stimulate the proliferation of antigen activated cells only, since its biological activity manifests itself resulting from interaction with a specific receptor expressed only by the activated target cells.

The claimed composition has immunomodulating action and can be used for the treatment of malignant diseases; various infections: sepsis, peritonitis, pancreatitis, osteomyelitis, pyodermatitis, endometritis, enteritis, mycosis; antibiotics-resistant infections: tuberculosis, pseudotuberculosis; hepatitis B and C, HIV-infection, Chlamydia; autoimmune diseases: multiple sclerosis, amyotrophic lateral sclerosis, glomerulonephritis, Crohn's disease, neurodermatitis; allergic diseases: grass pollen allergy, animal fur allergy, allergic asthma, contact allergy, dietary allergy, etc.

It is known that genetically modified microorganisms and, particularly, yeasts can be the producers of heterologous proteins. In particular, the methods of gene engineering and biotechnology allow obtaining yeast recombinant producers of the proteins having cytokine activity.

Most animal cytokines are secretory proteins. During secretion the natural cytokine proteins obtain their carbohydrate residue, thus becoming glycoproteins, achieve correct spatial configuration and biological activity and finally become soluble. It is also known that heterologous proteins are frequently present inside the cells of the producer microorganism in the form of "inclusion bodies". In the "inclusion bodies" the target recombinant proteins are in insoluble (hydrophobic) and inactive form. In particular, it is caused by the fact that heterologous proteins do not take the correct conformation during the synthesis inside the cells of the producer microorganism.

Various techniques are proposed (application US 20060052583, published at 09.03.2006) to solute the hydrophobic heterologous proteins in order to provide the proteins with proper conditions to take correct conformation. However, the present invention provides the use of the mentioned property of target proteins for producing a new composition. Hydrophobic recombinant proteins including recombinant interleukin-2 produced by yeasts tend to rest in the precipitate after centrifugation of destroyed producer cells (last paragraph at Page 4 of EP 0152358).

Thus, in context of the present invention, by "heterologous hydrophobic cytokine protein yeast producer" we mean a yeast strain, into which a cytokine encoding gene has been introduced; production of this protein is not regular for the considered yeast strain, therefore the protein is called heterologous. The invention relates only to the proteins that after being synthesized in the cells of the recombinant producer strain are then accumulated inside the cells and show hydrophobic properties. Despite the fact that under natural conditions the cytokines are glycosilated, secreted and hydrophilic, inside the producers microorganisms they can be accumulated inside the cells and consequently be non-glycosilated and hydrophobic. During the synthesis of such a recombinant protein by the producer cells, its spatial configuration is not properly formed, and the main characteristic of such proteins is that they remain in water insoluble fraction after destroyion of the producer cells.

Cytokine proteins are understood to be the growth factors that control proliferation, differentiation and function of blood cells and immune system (Sedlacek H. - H., Moroy T. "Immune reactions: headlines, overviews, tables and graphics". // Springer - Verlag Berlin Heidelberg. - 1995. - p. 1-53). Cytokines include interferons, colony-stimulating factors (CSF), chemokines, transforming growth factors, tumor necrosis factor, interleukins and some other factors (A.S.Simbirtsev. "Cytokines: classification and biological functions". //Cytokins and inflammation. - 2004. - Vol. 3, No. 2, p. 16-22).

To be more detailed, cytokines are small proteins (with molecular weight ranging from 8 to 80 kDa), acting autocrinally (i.e. acting at the cell which produces them) and/or paracrinally (i.e. acting at the neighboring cells). Cytokines are mainly secreted by the blood cells and by the cells of immune system and participate in the intercellular signal transduction within the immune system. To fulfill their function the cytokines shall bind to the specific receptors on membranes of their target cells and activate them. Cytokines that are synthesized by lymphocytes and are the regulators of proliferation and differentiation, particularly, of hematopoetic and immune system cells are also called lymphokines. They include interleukines (IL) (from *inter -* between and *leukins* - white blood cells), interferons and colony-stimulating factors.

The mechanism of action of one cytokine - interleukine-2 (IL-2) - includes stimulation of antigen-dependent proliferation of CD4+ and CD8+ lymphocytes. Other important properties of IL-2 are its influence on the Th1/Th2 balance, autocrine effect on Th1 cells and paracrine effect on the subpopulation of Th2 cells (Carol B. and Kendall A.S., 2002. Sedlacek H.-H., Moroy T. "Immune reactions: headlines, overviews, tables and graphics". // Springer-Verlag Berlin Heidelberg. - 1995. - p. 15-21). Influencing on the type to be selected and the duration of the immune response as well, IL-2 actually manifests many-sided immunobiological properties.

Therapeutic application of cytokines, and namely, such of IL-2, is being the subject of thorough studies during last decades. Great progress has been also achieved in obtaining recombinant cytokines in various producers.

Though *Saccharomyces cerevisiae* is the most widespread yeast producer, some other non-pathogenic yeasts can also be the producers, e.g., *Saccharomyces bulardi* or *Pichia* genus yeasts, e.g., *Pichia pastoris, Kluyveromyces* genus, e.g., *Kluyveromyces lactis,* or *Hansenula* genus, , e.g., *Hansenula polymorpha,* and the like.

Particularly, the *Saccharomyces cerevisiae* yeast strain deposited as No. Y-791 in the All-Russian Collection of Industrial Microorganisms can be taken as an example of yeast producers of human IL-2. Depending on the expression vector construction introduced into the producer strain, target heterologous proteins can be secreted or remain within the cells. If a protein remains within the cell, its further purification should be effected with destroyion of the producer strain cells.

Yeast cells can be destroyed by any known methods that ensure the integrity of the target protein, in particular, by physical methods, for example, utilizing a high-speed destroyor with glass beads, under high pressure, by freezing-melting method.

In this context, the integrity of the target product is ensured by adding protease inhibitors to the suspension of destroyed cells, so that the yeast proteases released during the destroyion of yeast cells should not destruct target protein, in particular, IL-2. Zymogenic methods of destroying yeast cells are also allowable if it is possible to avoid the proteolytic effect of the used agent on the target protein.

Water insoluble fraction can be separated by centrifugation. Thereby almost all water soluble components of yeast cells are transferred into the supernatant liquid, which is then removed, while water insoluble components remain in the precipitate. During the centrifugation the proteases, soluble proteins, ribonucleic acid (RNA), chromosome and plasmid DNA are eliminated. Separation of the water insoluble fraction can be done by other available methods, in particular, by filtration.

Separated water insoluble fraction can be somehow processed in order to improve its physical properties. For example, it can be once or several times rinsed with 0.005 M tris-HCl buffer solution or subjected to liophilization. This fraction can also be treated with acetone that leads to its complete dehydration and, after the acetone fraction is removed, the precipitate can be easily dried to obtain a powder-like mass.

In the dry precipitate of water insoluble fraction the percentage by weight of the target cytokine protein is 1-4%. Quantity of the cytokine protein may be determined by any standard method used for detecting the target protein, for example, by electrophoresis in polyacrylamide gel (Laemmli U. "Cleavage of structural proteins during the assembly of the head of bacteriophage T4". // Nature. - 1970. - 227. - p. 680-685) or by use of immune-enzyme analysis (Gehman L.O. and Robb R.J. "An ELISA-based assay for quantitation of human interleukin-2." // J. Immunol. Methods. - 1984. - vol.74. - p.39), etc.

The dose of the composition to be administered is determined basing on the activity of the cytokine obtained. For that purpose water insoluble fraction is solubilzed and activity of cytokine protein in liquid phase is determined by any standard test.

Special standard tests on evaluation of activity have been developed for each cytokine.

For example, the biological activity of the obtained IL-2 can be estimated by international standard test with use of the culture of IL-2-dependent tumor-specific cytotoxic mouse T-lymphocytes of CTLL-2 line (Hammerling U. et al. «Development and validation bioassay for interleukin-2». // J. Pharmaceut. & Biochem. Analysis, - 1992. - vol. 10. - p.547. Gearing A.J.H. and Thorpe R. «The international standard for human interleukin-2. Calibration by international collaborative study.» // J. Immunol. Methods. - 1984. - vol. 74. - p.39). Biologically active recombinant

IL-2 should stimulate the proliferation of these cells. Biological activity is determined by colorimetric method with tetrazolium dye MTT. The IL-2 stimulated viable cells absorb the dye and accumulate water insoluble dark blue crystals of formazan (reduced MTT) in cytoplasm. After the cultural medium is removed the crystals are completely dissolved in dimethyl sulfoxide, optical density of the obtained solution is measured by means of computer-aided photometer at the wave lengths of 530 and 690 nm. Dark blue coloration of solution in samples containing IL-2 should depend on concentration of IL-2 introduced in the cultural medium. By comparison of optical density of the sample and such of the activity standard of IL-2 the biological activity of IL-2 in the incubatory sample is determined. Particularly, as for IL-2, about 100 mg of dry water insoluble fraction show the activity level of 4-5 million International Units (IU). Thus, if it is necessary to use a dose of 1 million IU of IL-2, about 20-25 mg of dry water insoluble fraction is taken.

Addition of solubilzer and water to water insoluble fraction comprising heterologous hydrophobic cytokine protein leads to the conversion of this protein into its active form.

Solubilzer (detergent) is a substance that assisits the dissolution of hydrophobic substances in water. Sodium dodecyl sulphate, lauryl sarcosinate, deoxycholate, urea and

Triton-X100 can be used as solubilzers.

The recombinant IL-2 which is produced inside the yeast cells is water insoluble and it is linked to the cell wall. It has been found that solubilzer also allows detaching IL-2 from the cell wall.

Content of solubilzer in the composition may vary from 4.0% to 20.0% of dry weight of the water insoluble fraction, preferably, 12%.

The composition consisting of said water insoluble fraction and solubilzer represents an immunomodulating agent to which water shall be added before use.

Addition of water to the composition comprising dry water insoluble fraction and solubilzer leads to dissolution of the hydrophobic heterologous protein, achievement of the correct conformation of the heterologous protein and, therefore, to formation of the active cytokine. From 1 to 10 ml of water per 100 mg of water insoluble fraction may be added.

Composition may also additionally comprise any pharmaceutically acceptable excipients which are suitable for the formation of convenient pharmaceuetical form of said composition. These may be the excipients commonly used in pharmaceutical industry, stabilizers, antioxidants, etc.

Generally, the inventive composition may alternatively comprise, consist of, or essentially consist of any appropriate components disclosed in the present description, and also such inventive composition may additionally or alternatively be prepared in a way to exclude any component, material, ingredient or object which was used in a preparation known from the background art or which is not obligatory in terms of achieving the technical result of the invention.

### Example 1. Preparation of the composition.

For preparation of the composition an aliquot of the yeast strain *Saccharomyces cerevisiae Y-791* stored at -70⁰C in glycerin is taken and it is inoculated into nutrient medium Sd1 containing 50 mg/l of histidin. Composition of Sd1 medium is as follows: 0.67% yeast nitrogen base ("Difco Laboratories", Detroit, MI), 2% glucose. Cell mass to Sd1 medium volume ratio is approximately 0.2 g to1 liter.

Cultivation in Sdlmedium is carried out until the optical density OD₆₀₀ ranging from 2.5 to 4.0 (preferably, OD₆₀₀ =3.0) is achieved.

Upon reaching the required optical density value, the obtained inoculate is transferred in tenfold volume of Sd2 medium and further cultivated until OD₆₀₀ from 6.0 to 9.0 (preferably, OD₆₀₀ = 7.5) is achieved.

The composition of medium Sd2 is presented in Table 1.

**Table 1**

| Composition of nutrient medium Sd2 | |
|---|---|
| Medium component | Quantity per 1 liter |
| Histidine | 0.05 g |
| (NH₄)- citrate (anhydrous) | 7.6 g |
| MgSO₄ | 0.5 g |
| NaCl | 0.1 g |
| CaCl₂x6H₂O | 0.1g |
| KCl | 1.5g |
| KH₂PO₄ | 0.03g |
| Glucose | 20g |
| H₃BO₃ | 0.5mg |
| CuSO₄x5H₂O | 0.04 mg |
| KI | 0.1 mg |
| FeCl₃x6H₂O | 0.2 mg |
| MnSO₄ | 0.4 mg |
| NaMoO₄x2H₂O | 0.2 mg |
| ZnSO₄x7H₂O | 0.4 mg |
| Biotin | 0.02 mg |
| Calcium pantothenate | 2 mg |
| Folic acid | 0.002 mg |
| Inositol | 10 mg |
| Nicotinic acid | 0.4 mg |
| P-benzaminic acid | 0.2 mg |
| Pyridoxine hydrochloride | 0.4 mg |
| Riboflavin | 0.2 mg |
| Thiamine | 0.4 mg |

Cultivation in Sd2medium can be changed for cultivation in the same volume of PEP nutrition medium to achieve the same values of optical density. Composition of PEP nutrition medium is given in Table 2.

**Table 2**

| Composition of nutrient medium PEP | |
|---|---|
| Components | Content in PEP medium, g/l |
| Peptone | 20 |
| Glucose | 20 |

When cultivation in Sd2 medium (or in PEP medium) is completed, cells are separated from the cultural medium (by centrifugation or filtering) normally yielding 8-12 g of cells per 1 liter of nutrient medium. All further manipulations are performed with the cells, and the cultural medium is removed.

A 50% suspension of cells in buffer solution A with addition of PMSF (phenilmethylsulfonyl fluoride) is prepared with final concentration of 1 mM. Buffer solution A is a 0.005 M tris-HCl buffer solution, pH 7.2.

Yeast cells are destroyed with use of Dyno Mill destroyor at the temperature ranging from +2⁰C to +10⁰C and at the rotor rotation speed 3000 rpm.

Suspension of disrupted yeast cells is centrifuged at the temperature +4⁰C and rotor rotation speed from 4000 to 10000 rpm.

Supernatant fluid is removed and the precipitate is rinsed with buffer solution A to remove yeast proteases, RNA, chromosomal and plasmid DNA, soluble yeast proteins; the solution is then centrifuged again at the temperature +4⁰C and rotor rotation speed from 4000 to 10000 rpm. Supernatant is removed. Instead of centrifugation, water insoluble yeast fraction can be separated by filtration.

The wet precipitate obtained at this stage represents one of the embodiments of the invention. For therapeutic use, water and solubilzer must be added as described below. In such a way the composition of the invention may be obtained in liquid form.

Powder obtained from said destroyed cell precipitate dried by any available method, e.g. by freeze drying, represents another embodiment of the inventive composition. The amount of solubilzer of 4-20%, preferably, 12% by weight based on destroyed yeast dry product weight is added to the dried precipitate to obtain one more embodiment of the inventive composition.

Precipitate of destroyed cells can also be treated with cold acetone. In this case, more fine powder is obtained not requiring further grinding when mixing with solubilzer. For said processing a 75% suspension of the obtained disrupted cells sediment is prepared in water cooled to +4⁰C. For this purpose 0.3 ml of water is added to 1 g of precipitate. The suspension is prepared in an ice bath.

For further manipulations all glassware and acetone are cooled down to -10°C - -30°C, preferably, -20°C. The volume of the cooled acetone should exceed the volume of the cell suspension 15-20 times, preferably, 20 times.

Suspension of destroyed yeast cells is slowly introduced into acetone and homogenized for 5 minutes.

Disrupted dehydrated yeast cells are then separated from acetone by vacuum filtration at the temperature from -10°C to -30°C, preferably, -20°C, and rotation speed of 2000 - 5000 rpm.

The obtained destroyed yeast cells precipitate is rinsed with twofold to tenfold, preferably, fivefold volume of acetone and vacuum filtered again at the temperature of - 10°C to -30°C, preferably of -20°C, and rotation speed of 2000-5000 rpm.

Precipitate is transferred into an enamel vessel and dried in an exhaust hood at room temperature for 15-30 hours, preferably, 18 hours.

Finally, of 1 g of initial whole yeast cells about 80-160 mg of fine powder is obtained containing water insoluble fraction of destroyed IL-2 producer cells.

The powder obtained at this stage is one of the embodiments of the inventive composition. For therapeutic use water and solubilzer shall be added to it.

To obtain one more embodiment of the inventive composition, water and solubilzer in the amount of 4-20%, preferably, 12% by weight basing on powder weight shall be added to the powder.

In order to apply this composition comprising water insoluble fraction in the form of powder and solubilzer, it is necessary to stir 100 g of the obtained composition with 5 ml of water that corresponds to a dose of 4-5 million IU of IL-2.

### Example 2. Monoimmunotherapy for HIV-infection.

### Treatment Regimen

Treatment was performed in several courses. Each course lasted for 5 days and consisted in once daily administration of IL-2. Four courses were completed with 2-month intervals between them. In four weeks after each course, studies were performed including immunogram, clinical and biochemical blood test, viral HIV-load. First two courses were conducted with use of IL-2 Roncoleukin ^{®} for injections (Biotech, Russia), which represents a purified protein, in the amount of 1 million IU; during second two courses the inventive composition was administered orally in dose of 20 mg of composition per 5 ml of water, which corresponds to IL-2 dose of 1 million IU.

Patient: male, 38 years old. HIV-infection was diagnosed 4 months before the treatment with the inventive composition. Duration of the disease is unknown.

Before treatment:
- CD4+ lymphocytes blood count - 418 per microliter
- HIV-virus load - 41000 copies per ml
- urogenital Chlamydia.

Quantity of CD4+ T-lymphocytes shows the degree of HIV-initiated damage to immune system that has occurred so far. Regular repeatable measurements of blood plasma level of HIV-virus RNA and CD4+ T-lymphocytes blood counts are necessary for the determination of the disease progression risk in HIV-positive patient.

CD4+ blood count of less than 500/microliter and virus load of more than 10000 copies/ml are the indications for starting anti-virus therapy under asymptomatic HIV-infection.

### Results

The patient tolerated the treatment adequately; no complaints or adverse reactions were observed. Chlamidia infection has been completely resolved. The patient subjectively feels increased work ability.

**Table 3**

| CD4+ and HIV-viral Load Dynamics | | | | |
|---|---|---|---|---|
| Index | Before the treatment | After two courses of Roncoleukin^{®} | After the 3^{rd} course with the inventive preparation | After the 4^{th} course with the inventive preparation |
| CD4+ (units/microliter) | 418 | 480 | 756 | 1066 |
| HIV (copies/ml) | 41000 | 20000 | 1500 | 950 |

There are some data indicating that administration of IL-2 preparations in some cases increases virus load. Absence of such effect during the above disclosed treatment and even substantial decrease of virus load are the evidences of therapeutic perspective of said preparations.

Dynamics of both the first stage of treatment (with Ronkoleukin^{®}), and the second stage (with the inventive preparation) proved to be good.

Significant improvement of CD4+ contents dynamics under administration of the inventive preparation as compared to pure IL-2 for injections may be due to that the preparation possesses additional stimulating activity.

### Example 3. Monoimmunotherapy under HIV infection.

### Treatment Regimen

Treatment was performed in several courses. Each course lasted for 5 days and consisted in five once daily oral administrations of the inventive preparation in dose of 100 mg of preparation per 5 ml of water, which corresponds to IL-2 dose of 4-5 million IU. Two courses were completed with 4-month intervals between them.
Patient: male, 25 years old, HIV infection concomitant with persistent generalized lymphoadenopathy.

### Results

Immunograms were taken in 4 weeks after the completion of each course. Immunogram indicates relative CD3+, CD4+ 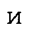 CD8+ lymphocytes blood counts (percentage of antigen positive cells content in blood T-lymphocytes subpopulation) and also immunoregulating index calculated as the ratio between CD4+ and CD8+ lymphocytes.

**Table 4**

| Dynamics of CD3+, CD4+ 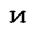 CD8+ and immunoregulating index | | | | |
|---|---|---|---|---|
| Index | Baseline at the beginning of the treatment | After 1^{st} course | After 2^{nd} course | Standard rate |
| CD3+ | 82% | 83% | 82% | 60-76% |
| CD4+ | 25% | 31% | 39% | 30-46% |
| CD8+ | 52% | 53% | 52% | 25-40% |
| CD4+/CD8+ | 0.48 | 0.6 | 0.75 | 1.0-1.7 |

Results of the immunogram indicate that treatment has resulted in improvement of patient's immune status, what decreased the patient's sensitivity to infectious agents and increased life quality.

### Example 4. Allergy Treatment

### Treatment Regimen

Treatment regimen was as follows: patient contacted with the allergic agent every other day; at the moment when allergic symptoms manifested themselves the patient was administered with the dose of the inventive composition of 100 mg of composition in 5 ml of water, which corresponds to IL-2 dose of 4-5 million IU.
1. Patient: Female, 36 years old, had been suffering from dietary allergy for 12 years. Dietary allergy was diagnosed on the basis of clinical manifestation and subcutaneous provocation tests. According to PRICK-test, reaction to 8 allergic agents has been detected.
   During said study the patient has been tested for single allergen - green apples. Treatment course lasted for 5 days according to the scheme outlined above; contact with green apples and administration of the composition of the invention was performed 3 times.
   Results
   On the 15^{th} day after the trial had started the PRICK-test was repeated. The test has shown the absence of the reaction to green apples and positive reaction to other 7 offending agents.
   A check test performed after 6 months has shown the same results.
2. Patient, 14 years old girl, had been suffering from cat fur allergy for 4 years. Clinical diagnosis was based on the observations made under direct contact with a cat. Treatment lasted for 5 days.
   Results:
   No allergic symptoms were detected during the contact with a cat on the 9^{th} day after the beginning of treatment.
   It is thus demonstrated that the composition of the invention possesses the therapeutic effect and can be efficiently used for treatment of a wide range of diseases, including those that have not been previously suggested to be treated by means of cytokine therapy.

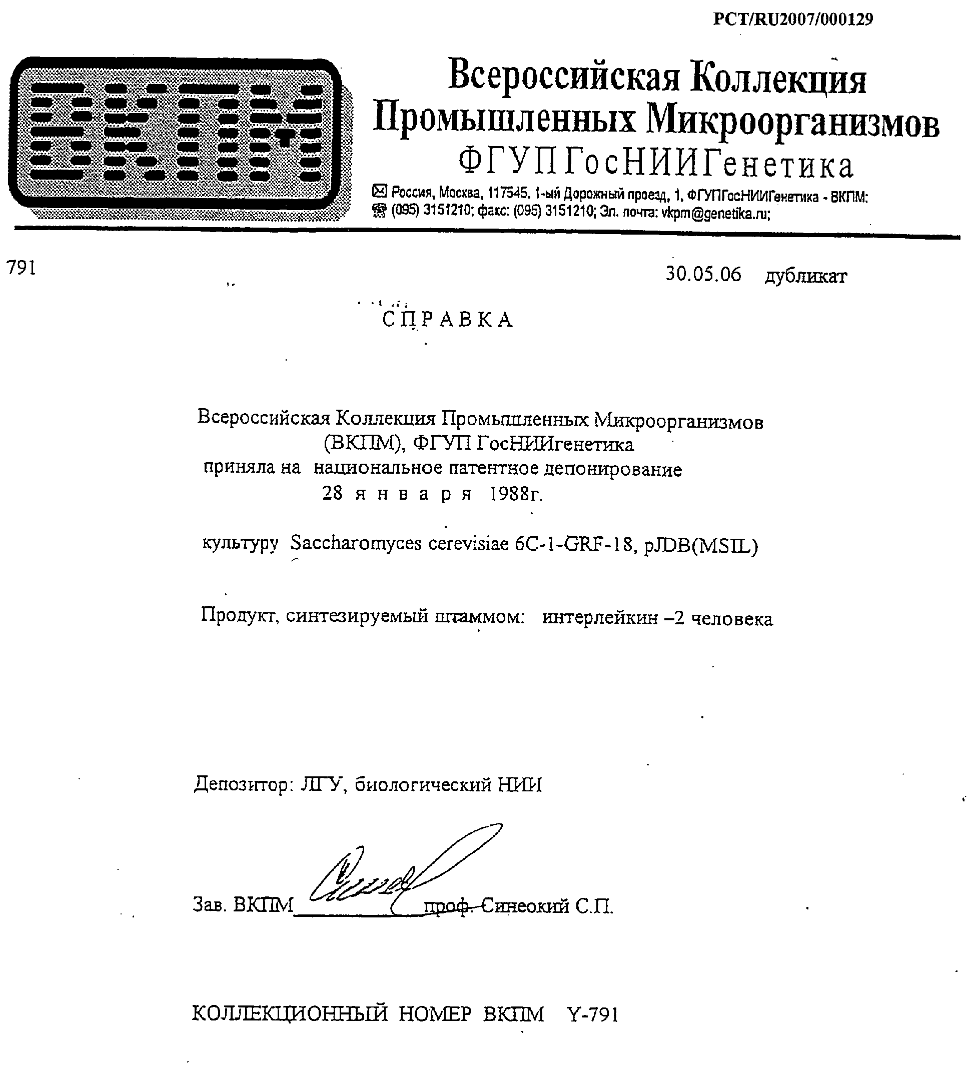
PCT/RU2007/000129


Всероссийская Коллекция
Промышленных Микроорганизмов
ФГУП ГосНИИГенетика
✉ Россия, Москва, 117545, 1-ый Дорожный проезд, 1, ФГУПГосНИИГенетика - ВКПМ;
☎ (095) 3151210; факс: (095) 3151210; Эл. почта: vkpm@genetika.ru;

791
30.05.06 дубликат

СПРАВКА

Всероссийская Коллекция Промышленных Микроорганизмов
(ВКПМ), ФГУП ГосНИИгенетика
приняла на национальное патентное депонирование
28 я н в а р я 1988г.

культуру Saccharomyces cerevisiae 6C-1-GRF-18, pJDB(MSIL)

Продукт, синтезируемый штаммом: интерлейкин –2 человека

Депозитор: ЛГУ, биологический НИИ

Зав. ВКПМ проф. Синеокий С.П.

КОЛЛЕКЦИОННЫЙ НОМЕР ВКПМ Y-791

## Claims

1. Immunomodulating composition comprising water insoluble destroyed cell fraction of yeast producer of heterologous hydrophobic cytokine protein, solubilzer and water, wherein in said producer a target hydrophobic cytokine protein has been synthesized.

2. The composition according to claim 1, **characterized in that** the cytokine protein is interleukin-2 (IL-2).

3. The composition according to claim 2, **characterized in that** the yeast producer is the *Saccharomyces cerevisiae* strain deposited as No. Y-791 in the All-Russian Collection of Industrial Microorganisms.

4. The composition according to claim 1-3, **characterized in that** the solubilzer is sodium dodecyl sulphate.

5. Composition for producing the immunomodulatiing composition according to claim 1, comprising water insoluble destroyed cell fraction of yeast producer of heterologous hydrophobic cytokine protein, wherein in said producer a target hydrophobic cytokine protein has been synthesized.

6. The composition according to claim 5, **characterized in that** the cytokine protein is interleukin-2 (IL-2).

7. The composition according to claim 6, **characterized in that** the yeast producer is the *Saccharomyces cerevisiae* strain, deposited as No. Y-791 in the All-Russian Collection of Industrial Microorganisms.

8. The composition according to claims 5-7, **characterized in that** it additionally comprises a solubilzer.

9. The composition according to claim 8, **characterized in that** the solubilzer is sodium dodecyl sulfate.
